(19) Europäisches Patentamt
European Patent Office
Office européen des brevets

(11) **EP 4 012 478 B1**

(12) **EUROPEAN PATENT SPECIFICATION**

(45) Date of publication and mention of the grant of the patent:
**01.07.2026 Bulletin 2026/27**

(21) Application number: **20862354.6**

(22) Date of filing: **07.09.2020**

(51) International Patent Classification (IPC):
*G02B 23/24* (2006.01)    *A61B 1/00* (2006.01)
*B29C 33/12* (2006.01)    *B29C 33/30* (2006.01)

(52) Cooperative Patent Classification (CPC):
**A61B 1/0011; A61B 1/00089;** B29C 33/12;
B29C 33/303; G02B 27/62

(86) International application number:
**PCT/JP2020/033716**

(87) International publication number:
**WO 2021/049444 (18.03.2021 Gazette 2021/11)**

(54) **FORMING MOLD FOR ENDOSCOPE HOOD**

FORMWERKZEUG FÜR ENDOSKOPHAUBE

MOULE DE FORMAGE POUR EMBOUT D'ENDOSCOPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2019 JP 2019165748**

(43) Date of publication of application:
**15.06.2022 Bulletin 2022/24**

(73) Proprietor: **Seed Co., Ltd.**
**Tokyo 113-8402 (JP)**

(72) Inventors:
• **KANEDA, Shiori**
**Tokyo 113-8402 (JP)**
• **MATSUNAGA, Toru**
**Tokyo 113-8402 (JP)**
• **SATO, Takao**
**Tokyo 113-8402 (JP)**
• **AKIMOTO Ryoji**
**Kawaguchi-shi, Saitama 332-0021 (JP)**
• **MORITA, Yoshinori**
**Kobe-shi, Hyogo 657-8501 (JP)**

(74) Representative: **Viering, Jentschura & Partner mbB**
**Patent- und Rechtsanwälte**
**Am Brauhaus 8**
**01099 Dresden (DE)**

(56) References cited:
WO-A1-2019/026473    JP-A- 2010 088 552
JP-A- 2010 088 552    JP-A- 2020 019 257
JP-A- H11 128 159    US-A1- 2008 102 151

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a molding die for molding an endoscope hood attached to an endoscope.

BACKGROUND ART

During the surgery or examination using an endoscope, a transparent hood is attached to an extension portion (insertion-side end portion) of the endoscope for the purpose of confirming an optimal distance from the extension portion of the endoscope to a biological tissue and of ensuring a field of view without obstacles. The hood is made of ABS, polycarbonate, vinyl chloride, silicone rubber, or the like.

However, such a hood is mounted for the purpose of confirming an optimum distance from the extension portion of the endoscope to the living tissue and ensuring a field of view without obstacles but a camera lens and an illumination lens are not covered by the hood. Thus, there is a problem that body fluid or oil may adhere and the field of view may become unclear during the examination or surgery using an endoscope on which the hood is mounted.

CITATION LIST

Patent Literature 1: Japanese Patent Application Laid-Open No. H11-047081; Patent Literature 2: JP 2010 088552 A

SUMMARY OF INVENTION

TECHNICAL PROBLEM

In order to solve such a problem, it is conceivable to form an endoscope hood from a hydrogel formed using a hydrophilic monomer. As a method for forming the endoscope hood from a hydrogel, a material containing the hydrophilic monomer is filled into a molding die made of, for example, plastic, and is subjected to a copolymerization reaction by raising a temperature. The material subjected to the copolymerization reaction is hydrated and swollen to obtain an endoscope hood made of a hydrogel. A shape of the endoscope hood obtained by the method corresponds to a shape of the molding die filled with the material containing the hydrophilic monomer. However, not only endoscopes having the same shape but also endoscopes having various shapes are provided. Therefore, it is necessary to manufacture different endoscope hoods using different molding dies depending on the shape of the endoscope.

As disclosed in Patent Document 1, an endoscope hood having a cylindrical exterior portion and a disk portion connected to an inner surface of the exterior portion may be provided. In such an endoscope hood, a shape of the disk portion is made different depending on a position of a camera lens or the like, of the endoscope, and an inner diameter of the exterior portion is made different depending on a diameter of a front end of the endoscope. That is, each of the disk portion and the exterior portion is required to be tailored, and versatility of the molding die cannot be improved. Patent Literature 2 discloses a molding die according to the preamble of claim 1.

Therefore, the present invention has been made to solve the problems described above, and an object of the present invention is to provide a molding die for endoscope hood capable of molding an endoscope hood having excellent antifogging and antifouling properties, enhancing versatility of the molding die, and further enhancing quality by making the endoscope hood with uniform thickness.

SOLUTION TO PROBLEM

In order to achieve the above object the present invention provides a molding die according to claim 1.

Further embodiments are described in the dependent claims.

ADVANTAGEOUS EFFECTS OF INVENTION

A molding die for endoscope hood according to the present invention includes a female mold, and a male mold. The male mold is inserted from an insertion port provided in the female mold. A predetermined filling space generated between the female mold and the male mold is filled with a material containing a hydrophilic monomer. The endoscope hood is molded by a copolymerization reaction of the material. Therefore, it is possible to mold an endoscope hood with excellent antifogging and antifouling properties even when used for a long time, and it is also possible to increase versatility of the molding die. The molding die further includes a groove-shaped portion protruding in a peripherally outward direction at any one of the female mold and the male mold, and a convex-shaped portion protruding in the peripherally outward direction at the other one. The male mold inserted into the female mold is positioned within the female mold by engagement between the groove-shaped portion and the convex-shaped portion. Therefore, the male mold can be reliably positioned within the female mold, the thickness of the endoscope hood can be made uniform, and the quality of the endoscope hood can be further improved. Such unique advantageous effects of the present invention are expected.

BRIEF DESCRIPTION OF DRAWINGS

Fig. 1 is a side cross-sectional view illustrating a molding die for endoscope hood according to a first embodiment of the present invention.
Fig. 2 is a side cross-sectional view illustrating the molding die, particularly a female mold.
Fig. 3 is a view illustrating the molding die, particularly the female mold; Fig. 3(a) is a plan view as viewed from a back end surface side, and Fig. 3(b) is a partial cross-sectional view on a front end surface side.
Fig. 4 is a side cross-sectional view illustrating the molding die, particularly a male mold.
Fig. 5 is a view illustrating the molding die, particularly the male mold; Fig. 5(a) is a plan view as viewed from the front end surface side, and Fig. 5(b) is a bottom view as viewed from the back end surface side.
Fig. 6 is a side cross-sectional view illustrating an endoscope hood obtained by the molding die.
Fig. 7 is a partial perspective view illustrating a state in which the endoscope hood obtained by the molding die is attached to an endoscope.
Fig. 8 is a view illustrating the endoscope hood obtained by the molding die; Fig. 8(a) is a cross-sectional view and Fig. 8(b) is a plan view.
Fig. 9 is a side cross-sectional view illustrating a molding die for endoscope hood according to a second embodiment of the present invention.
Fig. 10 is a side cross-sectional view illustrating an endoscope hood obtained by the molding die.
Fig. 11 is a partial perspective view illustrating a state in which the endoscope hood obtained by the molding die (the molding die shown in Fig. 9) is attached to an endoscope.

DESCRIPTION OF EMBODIMENTS

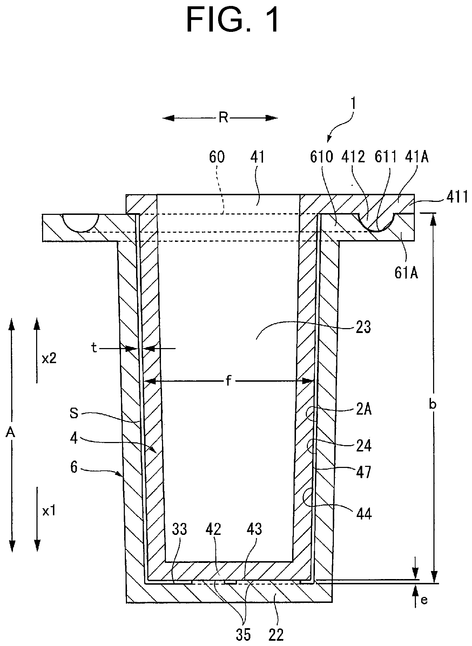

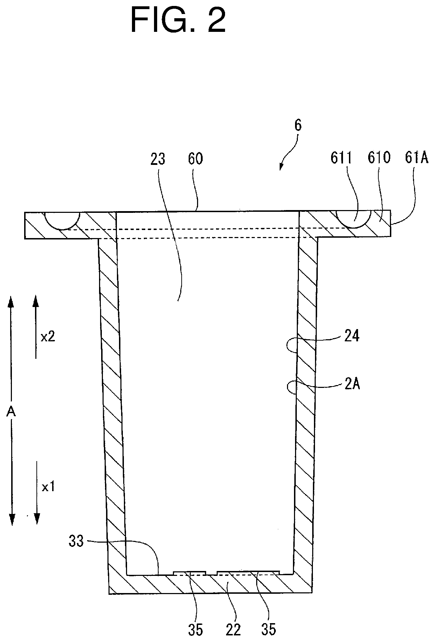

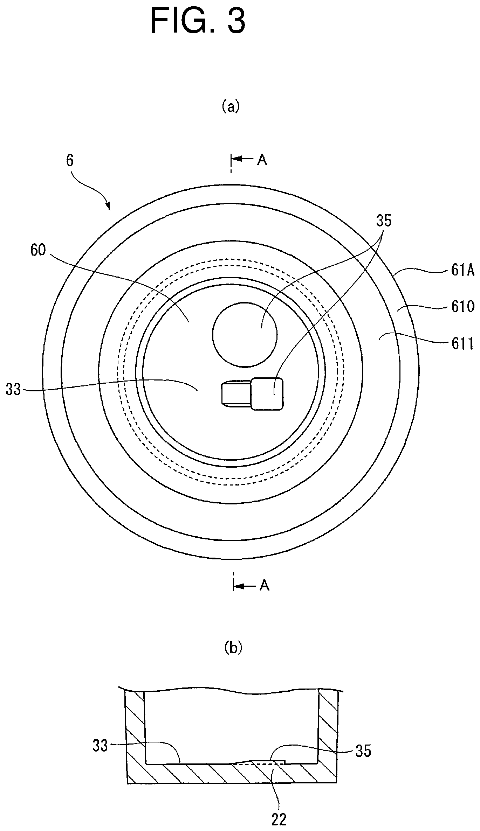

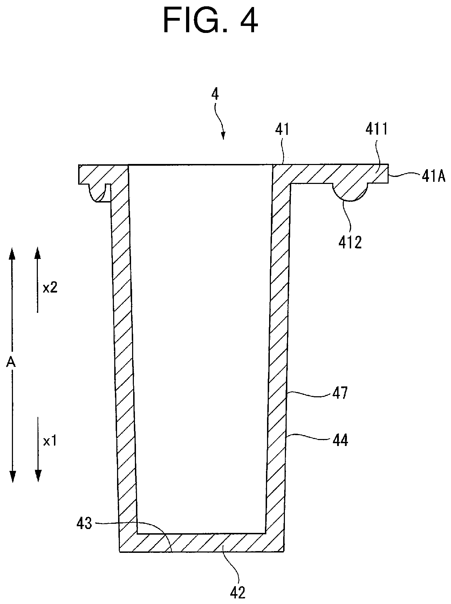

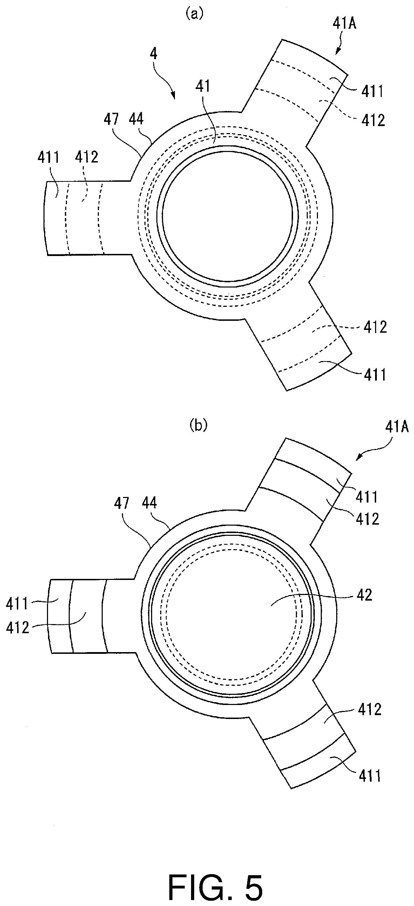

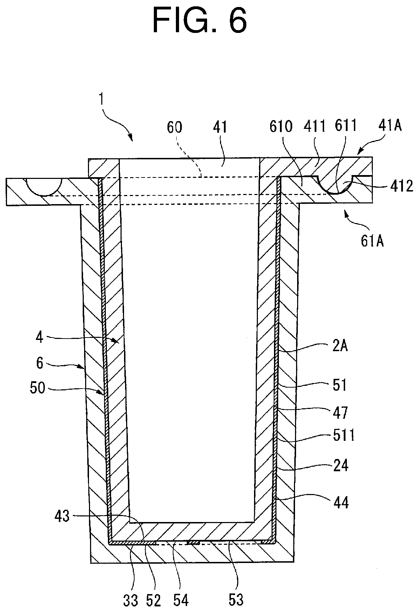

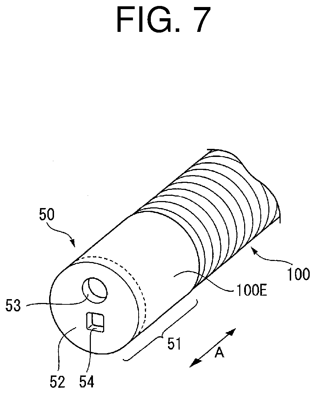

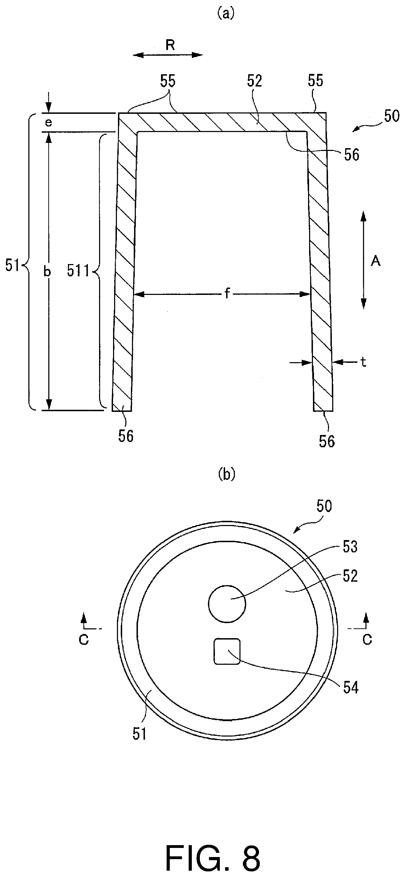

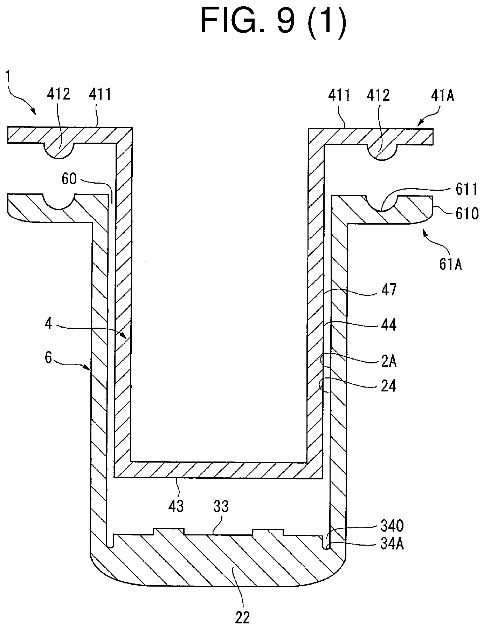

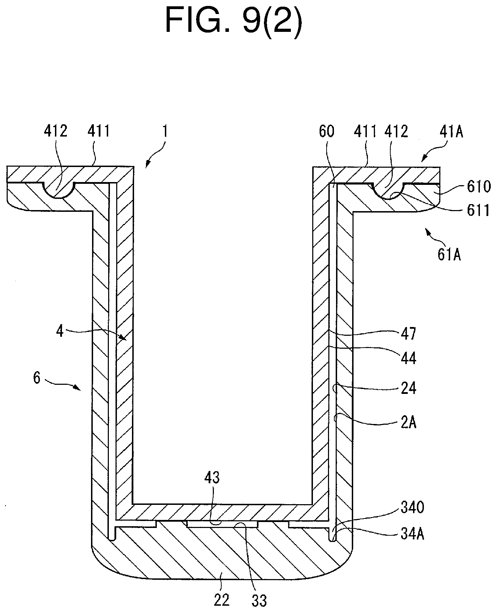

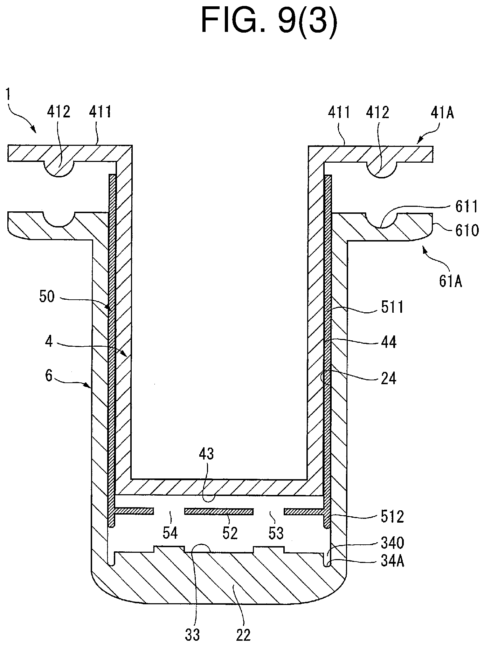

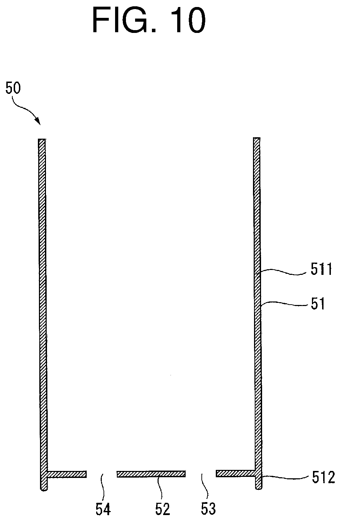

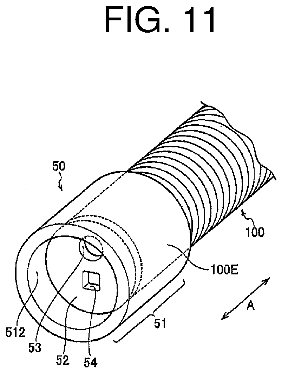

Hereinafter, embodiments will be described in detail with reference to the drawings as appropriate. However, unnecessarily detailed description may be omitted. For example, a detailed description of a well-known matter and a repeated description of substantially the same configuration may be omitted. This is to avoid unnecessary redundancy of the following description and to facilitate better understanding of those skilled in the art. Note that the inventor provides the accompanying drawings and the following description in order for those skilled in the art to fully understand the present disclosure, and does not intend to limit the subject matter described in the claims to the accompanying drawings and the following description.
In the following description of the drawings, the same or similar parts are denoted by the same or similar reference numerals. It should be noted that the drawings are schematic, and ratios of dimensions and the like may be different from actual ones. Therefore, specific dimensions and the like should be determined in consideration of the following description. In addition, the drawings may include parts having different dimensional relationships and ratios.
A molding die 1 for endoscope hood according to one embodiment will be described with reference to Figs. 1 and 2 to 5. Fig. 1 is a side cross-sectional view of the molding die 1 for endoscope hood (hereinafter, simply referred to as the "molding die 1"). Fig. 2 is a side cross-sectional view of the molding die 1, particularly a female mold. Fig. 3(a) is a plan view of the female mold, and Fig. 3(b) is a partial sectional view of the female mold. Fig. 4 is a side cross-sectional view of the molding die 1, particularly a male mold. Fig. 5(a) is a bottom view of the male mold, and Fig. 5(b) is a plan view of the male mold. Fig. 6 is a cross-sectional view illustrating an endoscope hood obtained by the molding die 1. Fig. 7 is a partial perspective view illustrating a state in which the endoscope hood is attached to an endoscope. Fig. 8 is a cross-sectional view and a plan view of the endoscope hood. Fig. 9 is a side cross-sectional view of a molding die 2 for endoscope hood (hereinafter, simply referred to as the "molding die 2"), which is a partial modification of the molding die 1. Fig. 10 is a cross-sectional view illustrating an endoscope hood obtained by the molding die 2. Fig. 11 is a partial perspective view illustrating a state in which the endoscope hood is attached to an endoscope.
The molding die 1 is a die for forming an endoscope hood attached to an insertion-side end portion (extension portion) of the endoscope. As illustrated in Fig. 7, an endoscope hood 50 is configured to be attached to an insertion-side end portion (extension portion) 100E of an endoscope 100.
In the present embodiment, the endoscope hood 50 is made of a hydrogel. Examples of the hydrogel include a hydrogel formed using only a hydrophilic monomer, and a hydrogel formed by adding a hydrophobic monomer, a crosslinkable monomer, or both to a hydrophilic monomer. The hydrophilic monomer contributes to the water content of the hydrogel, while the hydrophobic monomer contributes to the action of adjusting the water content and swelling rate of the hydrogel, and affects wettability and flexibility of the obtained endoscope hood 50. In addition, the crosslinkable monomer can control the density of polymer chains of the hydrogel depending on the content thereof, and can impart mechanical strength, shape stability, and solvent resistance to the hydrogel.

The water content of the hydrogel is not particularly limited as long as it can be molded, and can be, for example, 20 to 70 wt%.

Water content (wt%) = [(W-D)/W] $\times$ 100 (W: water-containing weight, D: dry weight)

The water content can be appropriately selected as necessary. For example, the water content of the exterior portion 51 and the water content of a disk portion 52 may be 20 to 70 wt%, respectively.

As the hydrophilic monomer, those having one or more hydrophilic groups in the molecule are preferable, and examples thereof include 2-hydroxyethyl (meth)acrylate, 2-hydroxymethyl (meth)acrylate, hydroxypropyl (meth)acrylate, glycerol (meth)acrylate, acrylamide, N,N-dimethyl (meth)acrylamide, N,N-diethyl (meth)acrylamide, N-vinylpyrrolidone, diacetone acrylamide, N-vinylacetamide, (meth) acrylic acid, (meth)acryloxyethyl succinic acid, itaconic acid, methacrylamidopropyl triammonium chloride, 2,3-dihydroxypropyl (meth)acrylate, and the like; and two or more hydrophilic monomers may be used in combination among these.

Among the hydrophilic monomers described above, 2-hydroxyethyl (meth)acrylate, N,N-dimethyl (meth)acrylamide, and N-vinylpyrrolidone are preferably used in the present invention from the viewpoint of handleability.

A blending ratio of the hydrophilic monomer is not particularly limited, but is preferably 50 wt% or more based on all the polymerized components because it affects the water content of the endoscope hood 50 to be obtained. When the blending ratio of the hydrophilic monomer is less than 50 wt%, the endoscope hood 50 having a sufficient water content cannot be obtained, which is not preferable since the antifouling and antifogging properties of the endoscope hood 50 may be deteriorated.

Examples of the hydrophobic monomer include siloxanyl (meth) acrylate, trifluoroethyl (meth)acrylate, methacrylamide, cyclohexyl (meth)acrylate, and normal butyl (meth) acrylate, and two or more of these hydrophobic monomers may be used in combination.

The hydrophobic monomer can change the water content of the endoscope hood 50 obtained according to a blending amount. However, when a blending ratio of the hydrophobic monomer is high, the water content is extremely lowered, and the flexibility of the endoscope hood 50 to be obtained is lowered. Therefore, for example, the blending ratio of the hydrophobic monomer is preferably less than 30 wt% with respect to the total amount of monomers.

Examples of the crosslinkable monomer include ethylene glycol di(meth)acrylate, methylene bisacrylamide, 2-hydroxy-1,3-dimethacryloxypropane, and trimethylolpropane triacrylate, and two or more of these may be used in combination.

The blending amount of the crosslinkable monomer is preferably 0.1 to 10 wt% with respect to the total amount of monomers from the viewpoint of the effect of adjusting the shape of the endoscope hood 50 to be obtained. When the blending amount is less than 0.1 wt%, a mesh structure of the endoscope hood 50 is insufficient, and when the blending amount exceeds 10 wt%, the mesh structure is conversely excessive, the endoscope hood 50 becomes brittle and has decreased flexibility.

Examples of a polymerization initiator to be used for polymerizing a mixture of the monomers stated above include peroxides such as lauroyl peroxide, cumene hydroperoxide and benzoyl peroxide which are general radical polymerization initiators; azobisvaleronitrile; and azobisisobutyronitrile. An addition amount of the polymerization initiator is preferably about 10 to 3500 ppm with respect to the total amount of monomers.

The endoscope hood 50 according to the present embodiment is formed into a desired shape after a single or a plurality of the monomers stated above are mixed, and is made of a single material.

As a step of obtaining the polymer, a monomer mixed solution obtained by mixing monomers as components is placed in the molding die of such as a metal, glass, or plastic and sealed, and a temperature is raised stepwise or continuously in a thermostatic bath or the like in a range of 25°C to 120°C. The copolymerization reaction is completed in 5 to 120 hours, whereby a molding die containing a polymer can be obtained. For polymerization, ultraviolet rays, electron beams, gamma rays, and the like can be used.

As a step of obtaining the hydrogel, the molding die after the polymerization is completed is cooled to room temperature, the polymer contained in the molding die is peeled off from the molding die, and cutting and polishing are performed as necessary. The polymer is hydrated and swollen to form the hydrogel. Examples of the liquid (swelling liquid) to be used include, but are not limited to, water, physiological saline, isotonic buffer, and a solution obtained by mixing an organic solvent such as ethanol with these. The swelling liquid is heated to 60°C to 100°C, and the polymer is immersed in the swelling liquid for a certain period of time to be brought into a swollen state. In addition, it is preferable to remove unreacted monomers adhering to the polymer during the swelling treatment. The obtained hydrogel can be shaped by being subjected to high-pressure steam sterilization at 110°C to 130°C for 10 to 60 minutes while being immersed in the swelling liquid such as physiological saline.

Since the endoscope hood 50 according to the present embodiment is excellent in the antifogging and antifouling properties, it is possible to easily remove body fluid and oil adhering to the surface of the hydrogel covering a lens portion with water ejected from a water supply mechanism provided for the endoscope 100 during the surgery or examination. It is also possible to suppress fogging. Therefore, even in the surgery or examination over a long period of time, it is not necessary to remove an endoscope 100 for removing contamination or cloudiness of the lens portion, and thus, it is possible to shorten the time of the surgery or examination and to reduce the burden on a patient.

As illustrated in Figs. 7 and 8, the endoscope hood 50 includes an exterior portion 51 and a disk portion 52. The endoscope hood 50 has an axial direction A and a radial direction R. The axial direction A of the endoscope hood 50 is along an insertion direction of the endoscope 100. The exterior portion 51 is provided in a circle in a direction perpendicular to an outer circumference of the disk portion 52. The exterior portion 51 has a cylindrical shape extending in the axial direction A, and may have a tapered shape that tapers toward the insertion direction of the endoscope 100. The exterior portion 51 abuts on the endoscope 100 and has a function of fixing the endoscope hood 50 to the endoscope 100. Therefore, an inner diameter f of the exterior portion 51 is appropriately designed according to the endoscope 100 to be used.

As illustrated in Fig. 8, the endoscope hood 50 includes a front end surface 55 located on a front end side of the endoscope hood 50 and an opposing surface 56 facing the front end surface 55. The front end surface 55 is a surface on the front end side in the insertion direction of the endoscope 100 to which the endoscope hood 50 is attached. The front end surface 55 includes an end edge of the exterior portion 51 in the axial direction A and an outer surface of the disk portion 52. The opposing surface 56 includes the end edge of the exterior portion 51 in the axial direction A and an inner surface of the disk portion 52.

A length b of the exterior portion 51 is not particularly limited as long as it is a length sufficient to fix the endoscope hood 50 to the endoscope 100. The length b of the exterior portion 51 is preferably 5 to 20 mm, and more preferably 10 to 15 mm. In this case, the length b of the exterior portion 51 is 20 mm. A thickness t of the exterior portion 51 is preferably 0.1 to 1.0 mm, and more preferably 0.3 to 0.5 mm. In this case, the thickness t of the exterior portion 51 is 0.20 mm. The length b and the thickness t of the exterior portion 51 are appropriately designed.

The disk portion 52 has a circular shape in a plan view. The disk portion 52 is located at a leading edge of the exterior portion 51 in the axial direction A and is continuous with an inner surface of the exterior portion 51. The disk portion 52 includes a forceps port 53 serving as an inlet and an outlet of forceps, and a water supply port 54 serving as an outlet of a water supply unit for cleaning the disk portion 52. The forceps port 53 and the water supply port 54 are openings penetrating the disk portion 52, and constitute an opening of the present invention. A thickness e of the disk portion 52 is preferably 0.01 to 1.0 mm, more preferably 0.05 to 0.7 mm, and most preferably 0.1 to 0.5 mm. When the thickness e of the disk portion 52 exceeds 1.0 mm, the field of view obtained through the camera lens is adversely affected. In a case where the thickness e of the disk portion 52 is smaller than 0.01 mm, the disk portion 52 has lower strength, which is not preferable since breakage during use is thus likely to occur. In this case, the thickness e of the disk portion 52 is 0.2 mm. The thickness e of the disk portion 52, position and shape of the forceps port 53, and position and shape of the water supply port 54 are appropriately designed according to a structure of the endoscope to be mounted.

Next, the molding die 1 for molding the endoscope hood 50 configured as described above will be described in detail with reference to Figs. 1 to 5. As illustrated in Fig. 1, the molding die 1 includes a female mold 6 and a male mold 4. The molding die 1 is a die for molding the endoscope hood 50 by inserting the male mold 4 from an insertion port 60 provided in the female mold, filling a predetermined filling space S generated between the female mold 6 and the male mold 4 with the material containing the hydrophilic monomer, and causing the material to undergo a copolymerization reaction. The molding die 1 may be made of a material suitable for raising a temperature at the time of copolymerization reaction, and is made of, for example, plastic. The molding die 1 has a cylindrical shape and has an axial direction A and a radial direction R. The axial direction A of the molding die 1 is along the axial direction A of the endoscope hood 50.

Each of the female mold 6 and the male mold 4 is made as a single piece. Therefore, the molding die 1 of the present embodiment includes two members, the female mold 6 and the male mold 4. The filling space S is surrounded by an inner surface of the female mold 6 and an outer surface of the male mold 4.

Since the molding die 1 has two molds (the female mold 6 and the male mold 4), a shape of the female mold 6 and a shape of the male mold 4 can be tailored according to the shape of the endoscope hood 50. When the shape of the endoscope hood 50 is adjusted, the female mold 6 and the male mold 4 may be adjusted respectively, only the male mold 4 may be adjusted without adjusting the female mold 6, or only the female mold 6 may be adjusted without adjusting the male mold 4. The number of combination patterns of the molds is increased, and thus the endoscope hood 50 can have the increased number of shape patterns. The versatility of each mold can be enhanced.

The molding die 1 includes a groove-shaped portion 61A protruding in a peripherally outward direction at any one of an edge portion of the insertion port 60 in the female mold 6 and a predetermined position corresponding to the insertion port 60 in the female mold 6 around the male mold 4 after being inserted into the female mold 6, and a convex-shaped portion 41A protruding in the peripherally outward direction at the other one to be engageable with the groove-shaped portion 61A. The male mold 4 inserted into the female mold 6 is positioned within the female mold 6 by engagement between the groove-shaped portion 61A and the convex-shaped portion 41A.

As illustrated in Figs. 2 and 3, the female mold 6 has a bottomed cylindrical shape, and has a housing portion 23 for housing the male mold 4 at the center in the radial direction. The housing portion 23 is a space surrounded by an inner surface 2A of the female mold 6, and has a front end closed in the axial direction A and a back end opened as the insertion port 60. The male mold 4 is configured to be inserted into the housing portion 23 from the insertion port 60 along the axial direction A. In this manner, the female mold 6 has the insertion port 60 having the opened back end for taking in and out the male mold 4, and a front end surface 22 opposite to the insertion port 60 in the axial direction A.

A first direction X1 in which the male mold 4 is inserted into the female mold 6 is a direction from the insertion port 60 at the back end toward the front end surface 22, and a second direction X2 in which the male mold 4 is taken out from the female mold 6 is a direction from the front end surface 22 toward the insertion port 60 at the back end. The front end surface 22 is located on a front end side in the insertion direction of the male mold 4 into the female mold 6. In the present embodiment, the first direction (the insertion direction of the male mold 4 into the female mold 6) X1 coincides with the insertion direction of the endoscope 100 to which the endoscope hood 50 molded by the molding die 1 is attached.

The inner surface 2A of the female mold 6 is an outer peripheral forming surface 24 corresponding to an outer peripheral surface of the endoscope hood 50. The inner surface 2A of the female mold 6 has a tapered shape that tapers toward the insertion direction of the endoscope 100 (the first direction X1 in the present embodiment). An inclination angle of the inner surface 2A of the female mold 6 with respect to the axial direction A may be 0.01° or more and 10.0° or less, preferably 0.01° or more and 5.0° or less, and more preferably 0.01° or more and 2.0° or less. The outer peripheral forming surface 24 is provided over the entire inner surface 2A of the female mold 6. The outer peripheral forming surface 24 is a surface surrounding the filling space S. The outer peripheral forming surface 24 is disposed at a distance from the outer surface (a mounting forming surface to be described later) of the male mold 4. The outer peripheral forming surface 24 is a surface corresponding to an outer peripheral surface of the exterior portion 51 of the endoscope hood 50.

An inner surface of the front end surface 22 of the female mold 6 is a front end forming surface 33 and is a surface corresponding to a front end surface 55 of the endoscope hood 50. The front end forming surface 33 is a surface corresponding to the outer surface of the disk portion 52 of the endoscope hood 50. The front end forming surface 33 is disposed at the center of the female mold 6 in the radial direction R and has a circular shape in plan view.

An opening forming portion 35 corresponding to the openings (the forceps port 53 and the water supply port 54) of the endoscope hood 50 is formed on the front end forming surface 33. The opening forming portion 35 protrudes in the axial direction A (the second direction X2 of the present embodiment) from the front end forming surface 33. In a region where the opening forming portion 35 is provided, the opening forming portion 35 of the female mold 6 and an opposite-forming surface 43 of the male mold 4 abut on each other, and an opening penetrating the disk portion 52 of the endoscope hood 50 is formed. In a region around the opening forming portion 35, the front end forming surface 33 of the female mold 6 and the opposite-forming surface 43 of the male mold 4 are disposed apart from each other, and the thick disk portion 52 is formed.

The positions and shapes of the openings of the endoscope hood 50 can be adjusted by position and shape of the opening forming portion 35. As another embodiment, the opening forming portion 35 may be provided in the male mold 4, or may be provided in both the female mold 6 and the male mold 4. By providing the opening forming portion 35 in any one of the female mold 6 and the male mold 4, it is sufficient to adjust only one of the female mold 6 and the male mold 4 when adjusting an opening pattern of the endoscope hood 50, and the versatility of the molding die 1 can be enhanced.

In addition, the groove-shaped portion 61A is provided in an entire edge portion (entire peripheral edge) of the insertion port 60 at the back end of the female mold and protrudes in the peripherally outward direction. The groove-shaped portion 61A has a flange 610 formed in a circular shape over the entire circumference at the edge portion of the insertion port 60 of the female mold 6, and a groove 611 is formed in one surface (surface facing the second direction X2) of the flange 610 facing the convex-shaped portion 41A to be described later. In this case, the groove 611 is continuously formed in a circular shape over the entire circumference substantially at the center in the radial direction of one surface of the flange 610. In this case, the groove 611 is formed in a substantially semicircular cross section or a substantially semi-cylindrical shape in cross section. The dimension from the center of the insertion port 60 of the female mold 6 to an outer peripheral edge of the flange 610 is 10 mm, and the dimension from the center of the insertion port 60 of the female mold 6 to the deepest portion of the groove 611 of the flange 610 is 8 mm.

As illustrated in Figs. 4 and 5, the male mold 4 is disposed in the axial direction A in the housing portion 23 of the female mold 6. The male mold 4 has a male back end 41 positioned on a side of the insertion port 60 at the back end of the female mold 6, that is, forming the edge portion (entire peripheral edge portion) of the insertion port 60, and a male front end 42 positioned on a front end surface side of the female mold 4. The opposite-forming surface 43 to be described later is formed at the male front end 42.

The male mold 4 has an outer surface surrounding the filling space S. The outer surface of the male mold 4 includes the opposite-forming surface 43 and a mounting forming surface 44. The opposite-forming surface 43 is a surface corresponding to the opposing surface 56 of the endoscope hood 50. The opposite-forming surface 43 is a surface corresponding to a surface of the disk portion 52 on a side of a mounting portion 511. The opposite-forming surface 43 is disposed at the center of the male mold 4 in the radial direction R and has a circular shape in a plan view.

The opposite-forming surface 43 of the male mold 4 is disposed at a distance from the front end forming surface 33 of the female mold 6 in the axial direction A. A distance e between the opposite-forming surface 43 and the front end forming surface 33 is the thickness e of the disk portion 52 of the endoscope hood 50. The thickness e of the disk portion 52 can be adjusted by adjusting the distance e between the opposite-forming surface 43 and the front end forming surface 33. Since the male mold 4 has the opposite-forming surface 43 and the female mold 6 has the front end forming surface 33, the front end surface 55 and the opposing surface 56 of the endoscope hood 50 and a region therebetween can be adjusted by adjusting any one of the female mold 6 and the male mold 4, and the versatility of the molding die 1 can be enhanced. As described above, the opposite-forming surface 43 abuts on the opening forming portion 35 of the female mold 6.

A surface extending in the axial direction A from the opposite-forming surface 43 to the male back end 41 constitutes the mounting forming surface 44. A distance b between the opposite-forming surface 43 and the male back end 41 in the axial direction A is a length of the mounting forming surface 44 in the axial direction A, which corresponds to a length b of the mounting portion 511 of the endoscope hood 50.

The mounting forming surface 44 is a surface corresponding to an inner peripheral surface of the mounting portion 511 of the endoscope hood 50. The mounting forming surface 44 is disposed to opposite to the outer peripheral forming surface 24 of the female mold 6. The filling space S sandwiched between the outer peripheral forming surface 24 of the female mold 6 and the mounting forming surface 44 of the male mold 4 is a mounting space corresponding to the mounting portion 511 of the exterior portion 51 of the endoscope hood 50. A distance t between the outer peripheral forming surface 24 and the mounting forming surface 44 is the width of the mounting space, and is a thickness t of the mounting portion 511 of the endoscope hood 50. The thickness t of the mounting portion 511 can be adjusted by adjusting the distance t between the outer peripheral forming surface 24 and the mounting forming surface 44. Furthermore, an inner diameter f of the mounting portion 511 can be adjusted by adjusting an outer diameter f of the mounting forming surface 44. The mounting forming surface 44 has a tapered shape that tapers toward the insertion direction of the endoscope 100 (the first direction X1 in the present embodiment). In a configuration in which the mounting forming surface 44 extends in parallel with the outer peripheral forming surface 24, the thickness of the mounting portion 511 of the endoscope hood 50 becomes constant. The mounting forming surface 44 may not have a tapered shape and may be along the axial direction A or may not be parallel to the outer peripheral forming surface 24.

In a configuration in which the thickness t of the mounting portion 511 varies, the distance between the outer peripheral forming surface 24 and the mounting forming surface 44 may vary.

The male mold 4 is provided with a convex-shaped portion 41A at a predetermined position corresponding to the insertion port 60 of the female mold 6 around the male mold 4 after being inserted into the female mold 6, and protrudes in the peripherally outward direction. The convex-shaped portion 41A has a plurality of plates 411 formed at predetermined intervals in the peripheral direction at an edge portion of the male back end 41 of the male mold 4, and a protruding portion 412 is formed on one surface of each plate 411 facing the groove-shaped portion 61A described above. In this case, the convex-shaped portion 41A is formed in a substantially quadrangular shape in which three plates 411 are slightly longer in the radial direction at intervals of 120° in the peripheral direction at the predetermined position around the male mold 4. Each protruding portion 412 is formed in an arc shape in a plan view and in a substantially semicircular cross section or a substantially semi-cylindrical cross section so as to fit into the groove 611 of the groove-shaped portion 61A at substantially the center of one surface of each plate 411. The dimension from the center of the male mold 4 concentrically inserted into the insertion port 60 of the female mold 6 at the insertion port 60 to the protruding portion 412 of each plate 411 is the same as the dimension from the insertion port 60 of the female mold 6 to the groove 611 of the flange 610.

When the endoscope hood 50 is manufactured by the molding die 1 configured as described above, as illustrated in Fig. 6, the material containing the hydrophilic monomer is injected into the female mold 6, and then the male mold 4 is inserted, and the material is subjected to the copolymerization reaction. In this case, after injecting the material into the female mold 6, the front end of the male mold 4 is inserted into the female mold 6 from the insertion port 60 at the back end of the female mold 6, and then the male mold 4 is inserted into the female mold 6. Since the inner surface 2A of the female mold 6 and an outer surface 47 of the male mold 4 have a tapered shape that tapers toward the front end side of the endoscope 100, the male mold 4 can be smoothly fitted into the female mold 6. The male mold 4 can also be withdrawn smoothly from the female mold 6. In addition, the male mold 4 is fitted into the female mold 6, and the groove-shaped portion 61A at the back end of the female mold 6 and the convex-shaped portion 41A at the back end of the male mold 4 are engaged with each other, such that the male mold 4 is fixed and held at a predetermined position in the female mold 6. That is, each protruding portion 412 having a substantially semicircular cross section of each plate 411 of the male mold 4 is smoothly fitted to an arbitrary position of the circular groove 611 having a substantially semicircular cross section of the flange 610 at the back end of the female mold 6. The dimension from the center of the insertion port 60 of the female mold 6 to the groove 611 of the flange 610 is the same as the dimension from the center of the male mold 4 inserted into the insertion port 60 of the female mold 6 at the insertion port 60 to the protruding portion 412 of each plate 411, such that the male mold 4 is concentrically disposed in the female mold 6. The disk portion 52 of the endoscope hood 50 is formed by a front end space sandwiched between the front end forming surface 33 of the female mold 6 and the opposite-forming surface 43 of the male mold 4, and the mounting portion 511 of the endoscope hood 50 is formed by the mounting space sandwiched between the outer peripheral forming surface 24 of the female mold 6 and the mounting forming surface 44 of the male mold 4. When the male mold 4 is concentrically disposed in the female mold 6 and position and angle of the male mold 4 are stabilized in the female mold 6, the endoscope hood 50 has a constant thickness and is formed to have a predetermined size and shape as a whole.

As described above, the molding die 1 for the endoscope hood has the female mold 6 and the male mold 4. The male mold 4 is inserted from the insertion port 60 provided in the female mold 6, the predetermined filling space generated between the female mold 6 and the male mold 4 is filled with the material containing the hydrophilic monomer, and the endoscope hood is molded by the copolymerization reaction of the material. Therefore, it is possible to mold the endoscope hood with excellent antifogging and antifouling properties even when used for a long time, and it is also possible to increase the versatility of the molding die. The molding die 1 includes the groove-shaped portion 61A protruding in the peripherally outward direction at the female mold 6, and the convex-shaped portion 41A protruding in the peripherally outward direction at the male mold 4. The male mold 4 inserted into the female mold 6 is positioned within the female mold 6 by engagement between the groove-shaped portion 61A and the convex-shaped portion 41A, thus it is possible to securely position the male mold 4 in the female mold 6, and the thickness of the endoscope hood can be made uniform to further improve the quality of the endoscope hood. The groove-shaped portion 61A has the flange 610 formed over the entire circumference at the edge portion of the insertion port 60 of the female mold 6. The groove 611 is formed and provided in one surface of the flange 610 facing the convex-shaped portion 41A. The convex-shaped portion 41A has the plurality of plates 411 formed at the predetermined intervals in the peripheral direction at predetermined positions around the male mold 4. The protruding portion 412 is formed and provided on one surface of each plate 411 facing the groove-shaped portion 61A. Therefore, a structure is simple, and the manufacturing cost can be reduced.

In this embodiment, the groove-shaped portion 61A and the convex-shaped portion 41A can be variously modified as follows.

(1) In the embodiment stated above, the groove-shaped portion 61A protrudes in the peripherally outward direction at the edge portion of the insertion port 60 of the female mold 6, and the convex-shaped portion 41A protrudes in the peripherally outward direction at the predetermined position corresponding to the insertion port 60 of the female mold 6 around the male mold 4 after being inserted into the female mold 6, such that the groove-shaped portion 61A and the convex-shaped portion 41A are engaged with each other. However, the groove-shaped portion may be protruded in the peripherally outward direction at a predetermined position around the male mold after being inserted into the female mold, and the convex-shaped portion may be protruded in the peripherally outward direction at the edge portion of the insertion port of the female mold, such that the groove-shaped portion and the convex-shaped portion are engaged with each other. Even in this case, the same effects as those of the embodiment stated above can be obtained.

(2) In the embodiment stated above, the groove-shaped portion 61A has the flange 610 formed over the entire circumference at the edge portion of the insertion port 60 of the female mold 6, the groove 611 is formed in one surface of the flange 610 facing the convex-shaped portion 41A, the convex-shaped portion 41A has the plurality of plates 411 (in this case, 3 plates) formed at the predetermined intervals (in this case, intervals of 120°) in the peripheral direction at the predetermined positions around the male mold 4, and the protruding portion 412 is formed on one surface of each plate 411 facing the groove-shaped portion 61A. However, in contrary, the groove-shaped portion may have the flange formed over the entire circumference at a predetermined position around the male mold, the groove may be formed in one surface of the flange facing the convex-shaped portion, the convex-shaped portion may have the plurality of plates formed at predetermined intervals in the peripheral direction at the edge portion of the insertion port of the female mold, and the protruding portion may be formed on one surface of each plate facing the groove-shaped portion. Even in this case, the same effects as those of the embodiment stated above can be obtained.

(3) In the embodiment stated above, the groove 611 is continuously formed in a circular shape over the entire circumference in one surface of the flange 610, and each protruding portion 412 is formed in an arc shape on one surface of each plate 411. However, the groove may be formed discontinuously in an arc shape or a dot shape partially in the peripheral direction in one surface of the flange, and each protruding portion may be formed in an arc shape or a dot shape on one surface of each plate. Even in this case, the same effects as those of the embodiment stated above can be obtained.

(4) In the embodiment stated above, the groove-shaped portion 61A has the flange 610 formed over the entire circumference at the edge portion of the insertion port 60 of the female mold 6, the groove 611 is formed in one surface of the flange 610 facing the convex-shaped portion 41A, the convex-shaped portion 41A has the plurality of plates 411 (in this case, 3 plates) formed at the predetermined intervals (in this case, intervals of 120°) in the peripheral direction at the predetermined positions around the male mold 4, and the protruding portion 412 is formed on one surface of each plate 411 facing the groove-shaped portion 61A. However, in contrary, the groove-shaped portion may have the plurality of plates (in this case, 3 plates) formed at predetermined intervals (in this case, intervals of 120°) in the peripheral direction at the edge portion of the insertion port of the female mold or at the predetermined positions around the male mold, the groove may be formed in one surface of each plate facing the convex-shaped portion, the convex-shaped portion may have the flange formed over the entire circumference at a predetermined position around the male mold or at the edge portion of the insertion port of the female mold, and the protruding portion may be formed on one surface of the flange facing the groove-shaped portion. In this case, the groove may be formed in an arc shape or a dot shape in one surface of the plate, and the protruding portion may be formed on one surface of the flange continuously in a circular shape over the entire circumference or discontinuously in an arc shape or a dot shape partially in the peripheral direction. Even in this case, the same effects as those of the embodiment stated above can be obtained.

(5) Further, both the groove-shaped portion and the convex-shaped portion may be formed of the flange or the plurality of plates, the groove-shaped portion may have the groove formed in one surface thereof, and the convex-shaped portion may have the protruding portion formed on one surface thereof. Even in this case, the same effects as those of the embodiment stated above can be obtained.

In this embodiment, exemplified is the molding die 1 for the endoscope hood 50 without the extension portion extending from the mounting portion 511 to the front end side in the insertion direction of the endoscope in the exterior portion 51. However, even in a case of the molding die 1 for the endoscope hood 50 with an extension portion 512 extending from the mounting portion 511 to the front end side in the insertion direction of the endoscope in the exterior portion 51, as illustrated in Fig. 10, the same effects as those of the embodiment stated above can be obtained as long as providing the groove-shaped portion 61A protruding in the peripherally outward direction at any one of the edge portion of the insertion port 60 in the female mold 6 and the predetermined position corresponding to the insertion port 60 in the female mold 6 around the male mold 4 after being inserted into the female mold 6, and the convex-shaped portion 41A protruding in the peripherally outward direction at the other one to be engageable with the groove-shaped portion 61A.

In this case, as illustrated in Fig. 9(1), the molding die 1 is different from the molding die 1 according to the embodiment stated above in that the front end surface 22 of the female mold 6 is formed thick, and a groove 340 having a predetermined depth is formed as an extension portion forming portion 34A on the extension of the outer peripheral forming surface 24 at the outermost peripheral portion of the front end forming surface 33. In this case, each of the outer peripheral forming surface 24 of the female mold 6 and the mounting forming surface 44 of the male mold 4 may have a tapered shape as in the embodiment stated above, or may be formed in a cylindrical shape.

When the endoscope hood 50 is manufactured by the molding die 1, similarly to the embodiment stated above, the material containing the hydrophilic monomer is injected into the female mold 6, and then the male mold 4 is inserted, and the material is subjected to the copolymerization reaction. In this case, as shown in Fig. 9(1), after the material is injected into the female mold 6, the front end of the male mold 4 is inserted into the female mold 6 from the insertion port 60 at the back end of the female mold 6, and the male mold 4 is inserted into the female mold 6. However, as shown in Fig. 9(2), the groove-shaped portion 61A at the back end of the female mold 6 and the convex-shaped portion 41A at the back end of the male mold 4 are engaged as the male mold 4 is fitted into the female mold 6, and the male mold 4 is fixed and held at a predetermined position in the female mold 6. That is, each protruding portion 412 having a substantially semicircular cross section of each plate 411 of the male mold 4 is smoothly fitted to an arbitrary position of the circular groove 611 having a substantially semicircular cross section of the flange 610 at the back end of the female mold 6. The dimension from the center of the insertion port 60 of the female mold 6 to the groove 611 of the flange 610 is the same as the dimension from the center of the male mold 4 inserted into the insertion port 60 of the female mold 6 at the insertion port 60 to the protruding portion 412 of each plate 411, such that the male mold 4 is concentrically disposed in the female mold 6. Then, as illustrated in Figs. 9(2) and 9(3), the disk portion 52 of the endoscope hood 50 is formed by the front end space sandwiched by the front end forming surface 33 of the female mold 6 and the opposite-forming surface 43 of the male mold 4, the mounting portion 511 of the endoscope hood 50 is formed by the mounting space sandwiched by the outer peripheral forming surface 24 of the female mold 6 and the mounting forming surface 44 of the male mold 4, and the extension portion 512 is formed on a front end side extension of the mounting space between the outer peripheral forming surface 24 of the female mold 6 and the mounting forming surface 44 of the male mold 4, that is, the extension portion forming portion 34A forming the groove 340 of the outermost peripheral portion of the front end forming surface 33 of the female mold 6. When the male mold 4 is concentrically disposed in the female mold 6 and position and angle of the male mold 4 are stabilized in the female mold 6, the endoscope hood 50 has a constant thickness and is formed to have a predetermined size and shape as a whole, as illustrated in Fig. 9(4).

As described above, the present invention has been described with reference to the embodiments, but it should be understood that the present invention is not limited to the description and drawings constituting a part of the disclosure in such embodiments. Various alternative embodiments, examples, and operations will be apparent to those skilled in the art from such disclosure.

REFERENCE SIGNS LIST

| | |
|---|---|
| 1 | molding die |
| 2A | inner surface |
| 23 | housing portion |
| 24 | outer peripheral forming surface |
| 3B | outer surface |
| 33 | front end forming surface |
| 35 | opening forming portion |
| 4 | male mold |
| 41A | convex-shaped portion |
| 41 | male back end |
| 411 | plate |
| 412 | protruding portion |
| 42 | male front end |
| 43 | opposite-forming surface |
| 44 | mounting forming surface |
| 50 | endoscope hood |
| 51 | exterior portion |
| 52 | disk portion |
| 53 | forceps port (opening) |
| 54 | water supply port (opening) |
| 55 | front end surface |
| 56 | opposing surface |
| 6 | female mold |
| 60 | insertion port |
| 61A | groove-shaped portion |
| 610 | flange |
| 611 | groove |
| 100 | endoscope |
| A | axial direction |
| S | filling space |
| X1 | first direction |
| X2 | second direction |

**Claims**

1. A molding die (1) for endoscope hood (50), comprising:

   a female mold (6); and
   a male mold (4), wherein
   the male mold (4) is configured to be inserted from an insertion port (60) provided in the female mold (6),
   a predetermined filling space (S) configured to be generated between the female mold (6) and the male mold (4) is configured to be filled with a material containing a hydrophilic monomer, wherein the endoscope hood (50) is moldable by a copolymerization reaction of the material,
   **characterized in that** the molding die (1) further includes:

      a groove-shaped portion (61A) protruding in a peripherally outward direction at any one of an edge portion of the insertion port (60) in the female mold (6) and a predetermined position corresponding to the insertion port (60) in the female mold (6) around the male mold (4) after insertion of the male mold (4) into the female mold (6), and
      a convex-shaped portion (41A) protruding in the peripherally outward direction at the other one to be engageable with the groove-shaped portion (61A), so that

   the male mold (4) inserted into the female mold (6) is positioned within the female mold (6) by engagement between the groove-shaped portion (61A) and the convex-shaped portion (41A), wherein

the groove-shaped portion (61A) has:

a flange (610) formed over entire circumference at the edge portion of the insertion port (60) in the female mold (6) or the predetermined position around the male mold (4), and
a groove (611) formed in one surface of the flange (610) facing the convex-shaped portion (41A).

2. The molding die (1) for endoscope hood (50) according to claim 1, wherein
the groove (611) is formed in the one surface of the flange (610) continuously in a circular shape over the entire circumference or discontinuously in an arc shape or a dot shape partially in a peripheral direction.

3. The molding die (1) for endoscope hood (50) according to claim 1, wherein
the groove-shaped portion (61A) has:

a plurality of plates (411) formed at predetermined intervals in the peripheral direction at the edge portion of the insertion port (60) in the female mold (6) or the predetermined position around the male mold (4), and
a groove (611) formed in one surface of each plate (411) facing the convex-shaped portion (41A).

4. The molding die (1) for endoscope hood (50) according to claim 3, wherein
the groove (611) is formed in an arc shape or a dot shape in the one surface of the plate (411).

5. The molding die (1) for endoscope hood (50) according to any one of claims 1 to 4, wherein
the convex-shaped portion (41A) has:

a plurality of plates (411) formed at predetermined intervals in the peripheral direction at the predetermined position around the male mold (4) or the edge portion of the insertion port (60) in the female mold (6), and
a protruding portion (412) formed on one surface of each plate (411) facing the groove-shaped portion (61A).

6. The molding die (1) for endoscope hood (50) according to claim 5, wherein
the protruding portion (412) is formed in an arc shape or a dot shape on the one surface of the plate (411).

7. The molding die (1) for endoscope hood (50) according to any one of claims 1 to 4, wherein

the convex-shaped portion (41A) has:
a flange (610) formed over entire circumference at the predetermined position around the male mold (4) or the edge portion of the insertion port (60) in the female mold (6), and
a protruding portion (412) formed on one surface of the flange (610) facing the groove-shaped portion (61A).

8. The molding die (1) for endoscope hood (50) according to claim 7, wherein
the protruding portion (412) is formed on the one surface of the flange (610) continuously in a circular shape over the entire circumference or discontinuously in an arc shape or a dot shape partially in a peripheral direction.


**Patentansprüche**

1. Eine Formvorrichtung (1) für eine Endoskophaube (50), die aufweist:

eine Negativform (6) und
eine Positivform (4), wobei
die Positivform (4) konfiguriert ist, um von einer in der Negativform (6) bereitgestellten Einführöffnung (60) eingeführt zu werden,
ein vorbestimmter Füllraum (S), der konfiguriert ist, um zwischen der Negativform (6) und der Positivform (4) erzeugt zu werden, konfiguriert ist, um mit einem Material, das ein hydrophiles Monomer enthält, gefüllt zu sein, wobei die Endoskophaube (50) mittels einer Copolymerisationsreaktion des Materials formbar ist,
**dadurch gekennzeichnet, dass** die Formvorrichtung (1) ferner aufweist:

einen nutförmigen Abschnitt (61A), der in einer peripheren Außenrichtung an irgendeinem/r von einem Randabschnitt der Einführöffnung (60) in der Negativform (6) oder einer vorbestimmten Position, die der Einführöffnung (60) in der Negativform (6) um die Positivform (4) herum nach Einführung der Positivform (4) in die Negativform (6) entspricht, vorsteht, und
einen konvex geformten Abschnitt (41A), der in der peripheren Außenrichtung an dem/r anderen vorsteht, um mit dem nutförmigen Abschnitt (61A) eingreifbar zu sein, so dass

die in die Negativform (6) eingeführte Positivform (4) durch Eingriff zwischen dem nutförmigen Abschnitt (61A) und dem konvex geformten Abschnitt (41A) innerhalb der Negativform (6) positioniert wird, wobei
der nutförmige Abschnitt (61A) hat:

einen Flansch (610), der über einen gesamten Umfang am Randabschnitt der Einführöffnung (60) in der Negativform (6) oder der vorbestimmten Position um die Positivform (4) herum gebildet ist, und
eine Nut (611), die in einer dem konvex geformten Abschnitt (41A) zugewandten Fläche des Flansches (610) gebildet ist.

2. Die Formvorrichtung (1) für eine Endoskophaube (50) gemäß Anspruch 1, wobei
die Nut (611) in der einen Fläche des Flansches (610) kontinuierlich in einer Kreisform über den gesamten Umfang oder diskontinuierlich in einer Bogenform oder einer Punktform teilweise in einer peripheren Richtung gebildet ist.

3. Die Formvorrichtung (1) für eine Endoskophaube (50) gemäß Anspruch 1, wobei
der nutförmige Abschnitt (61A) hat:

eine Mehrzahl von Platten (411), die in vorbestimmten Abständen in der peripheren Richtung am Randabschnitt der Einführöffnung (60) in der Negativform (6) oder an der vorbestimmten Position um das Positivformenelement (4) herum gebildet sind, und
eine Nut (611), die in einer dem konvex geformten Abschnitt (41A) zugewandten Fläche jeder Platte (411) gebildet ist.

4. Die Formvorrichtung (1) für eine Endoskophaube (50) gemäß Anspruch 3, wobei
die Nut (611) in der einen Fläche der Platte (411) in einer Bogenform oder einer Punktform gebildet ist.

5. Die Formvorrichtung (1) für eine Endoskophaube (50) gemäß irgendeinem der Ansprüche 1 bis 4, wobei
der konvex geformte Abschnitt (41A) hat:

eine Mehrzahl von Platten (411), die in vorbestimmten Abständen in der peripheren Richtung an der vorbestimmten Position um die Positivform (4) oder den Randabschnitt der Einführöffnung (60) in der Negativform (6) herum gebildet sind, und
einen vorstehenden Abschnitt (412), der an einer Fläche jeder Platte (411) gebildet ist, der dem nutförmigen Abschnitt (61A) zugewandt ist.

6. Die Formvorrichtung (1) für eine Endoskophaube (50) gemäß Anspruch 5, wobei
der vorstehende Abschnitt (412) in einer Bogenform oder einer Punktform auf der einen Fläche der Platte (411) gebildet ist.

7. Die Formvorrichtung (1) für eine Endoskophaube (50) gemäß irgendeinem der Ansprüche 1 bis 4, wobei

der konvex geformte Abschnitt (41A) hat:
einen Flansch (610), der über den gesamten Umfang an der vorbestimmten Position um die Positivform (4) oder den Randabschnitt der Einführöffnung (60) in der Negativform (6) herum gebildet ist, und
einen vorstehenden Abschnitt (412), der an einer dem nutförmigen Abschnitt (61A) zugewandten Fläche des Flansches (610) gebildet ist.

8. Die Formvorrichtung (1) für eine Endoskophaube (50) gemäß Anspruch 7, wobei
der vorstehende Abschnitt (412) an der einen Fläche des Flansches (610) kontinuierlich in einer Kreisform über den gesamten Umfang oder diskontinuierlich in einer Bogenform oder einer Punktform teilweise in einer peripheren Richtung gebildet ist.

**Revendications**

1. Matrice de moulage (1) pour capuchon d'endoscope (50), comprenant :

   un moule femelle (6) ; et
   un moule mâle (4), où
   le moule mâle (4) est configuré pour être inséré à partir d'un orifice d'insertion (60) prévu dans le moule femelle (6),
   un espace de remplissage prédéterminé (S) configuré pour être généré entre le moule femelle (6) et le moule mâle (4) est configuré pour être rempli d'un matériau contenant un monomère hydrophile, le capuchon d'endoscope (50) étant moulable par une réaction de copolymérisation du matériau,
   **caractérisée en ce que** la matrice de moulage (1) comprend en outre :

   une partie en forme de rainure (61A) faisant saillie dans une direction périphérique vers l'extérieur au niveau d'une quelconque parmi une partie de bord de l'orifice d'insertion (60) dans le moule femelle (6) et une position prédéterminée correspondant à l'orifice d'insertion (60) dans le moule femelle (6) autour du moule mâle (4) après insertion du moule mâle (4) dans le moule femelle (6), et
   une partie en forme convexe (41A) faisant saillie dans la direction périphérique vers l'extérieur au niveau de l'autre, de manière à pouvoir venir en prise avec la partie en forme de rainure (61A), de sorte que

   le moule mâle (4) inséré dans le moule femelle (6) soit positionné à l'intérieur du moule femelle (6) par mise en prise entre la partie en forme de rainure (61A) et la partie en forme convexe (41A), où
   la partie en forme de rainure (61A) comporte :

   une bride (610) formée sur toute la circonférence au niveau de la partie de bord de l'orifice d'insertion (60) dans le moule femelle (6) ou à la position prédéterminée autour du moule mâle (4), et
   une rainure (611) formée dans une surface de la bride (610) faisant face à la partie en forme convexe (41A).

2. Matrice de moulage (1) pour capuchon d'endoscope (50) selon la revendication 1, dans laquelle
   la rainure (611) est formée dans ladite une surface de la bride (610) de manière continue en forme circulaire sur toute la circonférence ou de manière discontinue en forme d'arc ou en forme de point partiellement dans une direction périphérique.

3. Matrice de moulage (1) pour capuchon d'endoscope (50) selon la revendication 1, dans laquelle
   la partie en forme de rainure (61A) comporte :

   une pluralité de plaques (411) formées à des intervalles prédéterminés dans la direction périphérique au niveau de la partie de bord de l'orifice d'insertion (60) dans le moule femelle (6) ou à la position prédéterminée autour du moule mâle (4), et
   une rainure (611) formée dans une surface de chaque plaque (411) faisant face à la partie en forme convexe (41A).

4. Matrice de moulage (1) pour capuchon d'endoscope (50) selon la revendication 3, dans laquelle
   la rainure (611) est formée en forme d'arc ou en forme de point dans ladite une surface de la plaque (411).

5. Matrice de moulage (1) pour capuchon d'endoscope (50) selon l'une quelconque des revendications 1 à 4, dans laquelle
   la partie en forme convexe (41A) comporte :

   une pluralité de plaques (411) formées à des intervalles prédéterminés dans la direction périphérique à la position prédéterminée autour du moule mâle (4) ou de la partie de bord de l'orifice d'insertion (60) dans le moule femelle (6), et
   une partie en saillie (412) formée sur une surface de chaque plaque (411) faisant face à la partie en forme de rainure (61A).

6. Matrice de moulage (1) pour capuchon d'endoscope (50) selon la revendication 5, dans laquelle
   la partie en saillie (412) est formée en forme d'arc ou en forme de point sur ladite une surface de la plaque (411).

7. Matrice de moulage (1) pour capuchon d'endoscope (50) selon l'une quelconque des revendications 1 à 4, dans laquelle
la partie en forme convexe (41A) comporte :

une bride (610) formée sur toute la circonférence à la position prédéterminée autour du moule mâle (4) ou de la partie de bord de l'orifice d'insertion (60) dans le moule femelle (6), et
une partie en saillie (412) formée sur une surface de la bride (610) faisant face à la partie en forme de rainure (61A).

8. Matrice de moulage (1) pour capuchon d'endoscope (50) selon la revendication 7, dans laquelle
la partie en saillie (412) est formée sur ladite une surface de la bride (610) de manière continue en forme circulaire sur toute la circonférence ou de manière discontinue en forme d'arc ou en forme de point partiellement dans une direction périphérique.

# FIG. 1

# FIG. 2

# FIG. 3

(a)

(b)

# FIG. 4

(a)

(b)

# FIG. 5

# FIG. 6

# FIG. 7

(a)

(b)

# FIG. 8

# FIG. 9 (1)

# FIG. 9(2)

# FIG. 9(3)

# FIG. 10

# FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11047081 A
- JP 2010088552 A